(19) Europäisches Patentamt
European Patent Office
Office européen des brevets

(11) **EP 1 025 073 B1**

(12) **EUROPÄISCHE PATENTSCHRIFT**

(45) Veröffentlichungstag und Bekanntmachung des Hinweises auf die Patenterteilung:
**13.02.2002 Patentblatt 2002/07**

(21) Anmeldenummer: **98964389.5**

(22) Anmeldetag: **05.10.1998**

(51) Int Cl.⁷: **C07C 45/33**, C07C 47/22, C07C 51/215, C07C 57/04, B01J 23/00

(86) Internationale Anmeldenummer:
**PCT/EP98/06301**

(87) Internationale Veröffentlichungsnummer:
**WO 99/20590 (29.04.1999 Gazette 1999/17)**

(54) **VERFAHREN DER HETEROGEN KATALYSIERTEN GASPHASENOXIDATION VON PROPAN ZU ACROLEIN UND/ODER ACRYLSÄURE**

METHOD OF HETEROGENEOUS CATALYZED VAPOR-PHASE OXIDATION OF PROPANE TO ACROLEIN AND/OR ACRYLIC ACID

PROCEDE D'OXYDATION EN PHASE GAZEUSE CATALYSEE HETEROGENE DU PROPANE EN ACROLEINE ET/OU EN ACIDE ACRYLIQUE

(84) Benannte Vertragsstaaten:
**BE DE DK ES FR GB IT NL**

(30) Priorität: **21.10.1997 DE 19746210**
**23.10.1997 DE 19746667**
**19.11.1997 DE 19751046**
**04.12.1997 DE 19753817**
**20.02.1998 DE 19807079**

(43) Veröffentlichungstag der Anmeldung:
**09.08.2000 Patentblatt 2000/32**

(73) Patentinhaber: **BASF AKTIENGESELLSCHAFT**
**67056 Ludwigshafen (DE)**

(72) Erfinder:
• **JACHOW, Harald**
**D-64625 Bensheim (DE)**
• **TENTEN, Andreas**
**D-67487 Maikammer (DE)**
• **UNVERRICHT, Signe**
**D-68169 Mannheim (DE)**
• **ARNOLD, Heiko**
**D-68159 Mannheim (DE)**

(56) Entgegenhaltungen:
EP-A- 0 575 897    DE-A- 19 622 331
FR-A- 2 756 499    GB-A- 1 340 891
GB-A- 1 398 385    US-A- 2 625 519
US-A- 5 191 116    US-A- 5 198 580

• DATABASE WPI Section Ch, Week 9018 Derwent Publications Ltd., London, GB; Class A41, AN 90-135680 XP002097342 & JP 02 083348 A (MITSUBISHI PETROCHEMICAL CO LTD), 23. März 1990

Bemerkungen:
Die Akte enthält technische Angaben, die nach dem Eingang der Anmeldung eingereicht wurden und die nicht in dieser Patentschrift enthalten sind.

## Beschreibung

Vorliegende Erfindung betrifft ein Verfahren der heterogen katalysierten Gasphasenoxidation von Propan zu Acrolein und/oder Acrylsäure, bei dem man ein aus Propan, molekularem Sauerstoff und gegebenenfalls Inertgas bestehendes Reaktionsgasausgangsgemisch bei einer Temperatur von 300 bis 500°C über einen Festbettkatalysator führt.

Acrolein und Acrylsäure sind bedeutende Zwischenprodukte, die beispielsweise im Rahmen der Herstellung von Wirkstoffen und Polymerisaten Verwendung finden.

Das gegenwärtig überwiegend angewandte Verfahren zur großtechnischen Herstellung von Acrolein und/ oder Acrylsäure bildet die gasphasenkatalytische Oxidation von Propen (z.B. EP-A 575 897), wobei das Propen überwiegend als Nebenprodukt der Ethylenherstellung durch Steamcracken von Naphta erzeugt wird.

Da die sonstigen Anwendungsgebiete des Propens, z.B. die Herstellung von Polypropylen, sich immer weiter ausdehnen, wäre es vorteilhaft, über ein großtechnisch anwendbares, wettbewerbsfähiges, Verfahren zur Herstellung von Acrolein und/oder Acrylsäure zu verfügen, dessen Rohstoffbasis nicht Propen sondern das z.B. als Erdgasbestandteil in großen Mengen natürlich vorkommende Propan ist.

In der EP-A 117146 wird vorgeschlagen, Acrylsäure aus Propan dadurch herzustellen, daß man in einer ersten Stufe Propan durch heterogene katalytische Dehydrierung unter Ausschluß von molekularem Sauerstoff in einen Propylen enthaltenden Produktstrom überführt und diesen zur Oxidation des darin enthaltenen Propens mittels molekularem Sauerstoff zu Acrolein und/oder Acrylsäure in nachfolgenden Oxidationsstufen über geeignete Oxidationskatalysatoren leitet.

Nachteilig an dieser Verfahrensweise ist, daß sie in notwendiger Weise mehrerer Reaktionsstufen bedarf, wobei die einzelnen Reaktionstufen bei unterschiedlichen Reaktionsbedingungen verwirklicht werden müssen.

Weiterhin ist die vorgenannte Verfahrensweise insofern von Nachteil, als der für die nicht oxidative Dehydrierung das Propans benötigte Katalysator durch Kohlenstoffablagerungen relativ rasch desaktiviert und regeneriert werden muß. Da das Dehydrierproduktgemisch auch Wasserstoff enthält, bezweifelt die CN-A 1105352 ferner die Möglichkeit einer unmittelbaren Weiterleitung des Dehydrierproduktgemisches in eine nachfolgende Oxidationsstufe.

Die CN-A 1105352 sowie Y. Moro-oka in Proceedings of the 10th International Congress on Catalysis, 19 - 24 July, 1992, Budapest, Hungary, 1993, Elsevier Science Publishers B. V., S. 1982 bis 1986, empfehlen, Propan zunächst in einer homogenen oxidativen Dehydrierung partiell in Propen zu wandeln und dieses ohne vorherige Abtrennung in anschließenden heterogen katalysierten Oxidationsstufen in Acrolein und/oder Acrylsäure überzuführen. Nachteilig an dieser Verfahrensweise ist, daß einerseits auch im Rahmen einer homogenen oxidativen Dehydrierung von Propan zu Propen Kohlenstoffbildung einhergeht und daß auch die Selektivität der Wertproduktbildung (Acrolein und/oder Acrylsäure) bei einer solchen Verfahrensweise nicht zu befriedigen vermag. So beträgt in der CN-A 1105352 die durch homogene oxidative Dehydrierung erzielte Selektivität der Propenbildung in den Ausführungsbeispielen bereits nur ≤40 Vol.-% und auch Moro-oka ist auf Selektivitäten der Acroleinbildung, bezogen auf umgesetztes Propan, von 64 mol-% beschränkt.

Es wurde auch schon vorgeschlagen, eine heterogen katalysierte oxidative Dehydrierung von Propan (mit ihr geht nicht notwendigerweise Kohlenstoffbildung einher) mit einer nachfolgenden heterogen katalysierten Oxidation des so erzeugten Propens zu Acrolein und/Acrylsäure zu koppeln (z.B. 210th ACS National Meeting, Chicago, August 20 - 24, 1995 oder WO 97/36849). Nähere Angaben über die Art und Weise der Kopplung (in der Regel erfordern beide Reaktionsschritte nicht miteinander vereinbare Reaktionsbedingungen) wurden jedoch nicht gemacht. Die CN-A 1105352 rät sogar dezidiert von einer solchen Kopplung ab, da die bei vernünftigen Propanumsätzen erreichbaren Selektivitäten der Propenbildung bei einer heterogen katalysierten oxidativen Dehydrierung nicht über diejenigen bei einer homogenen oxidativen Dehydrierung hinausgingen.

In Topics in Catalysis 3(1996) wird auf S. 265 bis 275 über die heterogen katalysierte oxidative Dehydrierung von Propan zu Propen an Cobalt- und Magnesiummolybdaten berichtet. Nachteilig an der Verfahrensweise der vorgenannten Referenz ist, daß sie, vermutlich zur Gewährleistung einer befriedigenden Selektivität der Propenbildung, in hoher Verdünnung durchgeführt wird, d.h., daß das Propan und molekularen Sauerstoff enthaltende Reaktionsgasausgangsgemisch zu 75 Vol.-% aus molekularem Stickstoff (Inertgas) besteht. Ein solch hoher Inertgasanteil vermag nicht zur Kopplung mit einer nachfolgenden Propenoxidation anzuregen, mindert er doch die bei einfachem Durchgang erzielbaren Acrolein- und/oder Acrylsäure-Raum-Zeit-Ausbeuten. Ferner erschwert ein solcher Stickstoffanteil eine im Anschluß an die Propenoxidation gegebenenfalls beabsichtigte Rückführung von nicht umgesetztem Propan und/oder Propen nach erfolgter Acrolein- und/oder Acrylsäureabtrennung.

In Journal of Catalysis 167, 550 - 559 (1997) wird ebenfalls über die heterogen katalysierte oxidative Dehydrierung von Propan zu Propen an Molybdaten berichtet. Nachteilig an der Verfahrensweise dieser Referenz ist, daß sie ebenfalls die Verwendung eines Reaktionsgasausgangsgemisches empfiehlt, dessen Anteil an molekularem Stickstoff 70 Vol.-% beträgt. Ferner fordert vorgenannte Referenz eine Dehydriertemperatur von 560°C. Eine solch hohe Dehydriertemperatur vermag ebenfalls nicht zur Kopplung mit einer nachfolgenden heterogen katalysierten Propenoxidation anzuregen, da sie die zu einer oxidativen Propenumwandlung zu Acrolein und/oder Acrylsäure üblicherweise eingesetzten Multimetalloxidaktivmassen schädigt.

In Journal of Catalysis 167, 560 - 569 (1997) wird ebenfalls eine Dehydriertemperatur von 560°C für eine heterogen katalysierte oxidative Dehydrierung empfohlen. In entsprechender Weise empfiehlt auch die DE-A 19530454 oberhalb von 500°C liegende Temperaturen für eine heterogen katalysierte oxidative Dehydrierung von Propan zu Propen.

Desweiteren wird in der Literatur über Versuche einer heterogen katalysierten Direktoxidation von Propan zu Acrolein und/oder Acrylsäure berichtet (z.B. Tagungsband, 210th ACS National Meeting, Chicago, August 20 - 24, 1995, FR-A 2693384 sowie 3rd World Congress on Oxidation Catalysis, R.K. Grasselli, S.T. Oyama, A.M. Gaffney and J.E. Lyons (Editors), 1997 Elsevier Science B.V., S. 375 - 382), doch vermag auch in diesen Arbeiten entweder die berichtete Selektivität der Acrolein- und/oder Acrylsäurebildung und/oder die berichte Ausbeute an Acrolein- und/oder Acrylsäure bei einfachem Durchgang nicht zu befriedigen.

Die EP-B 608838 betrifft ebenfalls die heterogen katalysierte Direktoxidation von Propan zu Acrylsäure. Nachteilig an der EP-B 608838 ist jedoch, daß die in dieser Schrift beispielhaft berichteten Selektivitäten der Acrylsäurebildung nicht nacharbeitbar sind. So ergaben diesseitige Nacharbeitungen eine verschwindende Selektivität der Acrylsäurebildung. Statt dessen wurde bei der Nacharbeitung dieser Beispiele Acroleinbildung gefunden, wobei die Selektivität der Acroleinbildung jedoch ≤30 mol.-% betrug.

Die Aufgabe der vorliegenden Erfindung bestand daher darin, ein Verfahren der heterogen katalysierten Gasphasenoxidation von Propan zu Acrolein und/oder Acrylsäure, bei dem man ein aus Propan, molekularem Sauerstoff und gegebenenfalls Inertgas bestehendes Reaktionsgasausgangsgemisch bei einer Temperatur von 300 bis 500°C über einen Festbettkatalysator führt, zur Verfügung zu stellen, das die Nachteile der im Stand der Technik beschriebenen und/oder empfohlenen Verfahrensweisen nicht aufweist.

Demgemäß wurde ein Verfahren der heterogen katalysierten Gasphasenoxidation von Propan zu Acrolein und/oder Acrylsäure, bei dem man ein aus Propan, molekularem Sauerstoff und gegebenenfalls Inertgas bestehendes Reaktionsgasausgangsgemisch bei einer Temperatur von 300 bis 500°C über einen Festbettkatalysator führt, gefunden, das dadurch gekennzeichnet ist, daß der Festbettkatalysator aus zwei räumlich aufeinanderfolgend angeordneten Katalysatorschüttungen A, B besteht, mit der Maßgabe, daß die Aktivmasse der Schüttung A wenigstens ein Multimetalloxid der allgemeinen Formel I

$$M^1_a Mo_{1-b} M^2_b O_x$$

mit

M$^1$ =    Co, Ni, Mg, Zn, Mn und/oder Cu, vorzugsweise Co, Ni und/oder Mg, besonders bevorzugt Co und/oder Ni,

M$^2$ =    W, V, Te, Nb, P, Cr, Fe, Sb, Ce, Sn und/oder La, vorzugsweise Sn, W, P, Sb und/oder Cr, besonders bevorzugt W, Sn und/oder Sb,

a =    0,5 bis 1,5, vorzugsweise 0,7 bis 1,2, besonders bevorzugt 0,9 bis 1,0,

b =    0 bis 0,5, vorzugsweise >0 bis 0,5 und besonders bevorzugt 0,01 bis 0,3 sowie

x =    eine Zahl, die durch die Wertigkeit und Häufigkeit der von Sauerstoff verschiedenen Elemente in I bestimmt wird,

und die Aktivmasse der Schüttung B wenigstens ein Multimetalloxid der allgemeinen Formel II

$$Bi_a, Mo_b, X^1_c, X^2_d, X^3_e, X^4_f, X^5_g, X^6_h, O_x, \qquad \text{(II)},$$

mit

X$^1$ =    W, V und/oder Te, vorzugsweise W und/oder V
X$^2$ =    Erdalkalimetall, Co, Ni, Zn, Mn, Cu, Cd, Sn und/oder Hg, vorzugsweise Co, Ni, Zn und/oder Cu, besonders bevorzugt Co, Ni und/oder Zn,
X$^3$ =    Fe, Cr und/oder Ce, vorzugsweise Fe und/oder Cr,

$X^4$= P, As, Sb und/oder B, vorzugsweise P und/oder Sb,

$X^5$= Alkalimetall, Tl und/oder Sn, vorzugsweise K und/oder Na,

$X^6$= seltenes Erdmetall, Ti, Zr, Nb, Ta, Re, Ru, Rh, Ag, Au, Al, Ga, In, Si, Ge, Th und/oder U, vorzugsweise Si, Zr, Al, Ag, Nb und/oder Ti,

a'= 0,01 bis 8, vorzugsweise 0,3 bis 4 und besonders bevorzugt 0,5 bis 2,

b'= 0,1 bis 30, vorzugsweise 0,5 bis 15 und besonders bevorzugt 10 bis 13,

c'= 0 bis 20, vorzugsweise 0,1 bis 10 und besonders bevorzugt 0,5 bis 3,

d'= 0 bis 20, vorzugsweise 2 bis 15 und besonders bevorzugt 3 bis 10,

e'= 0 bis 20, vorzugsweise 0,5 bis 10 und besonders bevorzugt 1 bis 7,

f'= 0 bis 6, vorzugsweise 0 bis 1,

g'= 0 bis 4, vorzugsweise 0,01 bis 1,

h'= 0 bis 15, vorzugsweise 1 bis 15 und

x'= eine Zahl, die durch die Wertigkeit und Häufigkeit der von Sauerstoff verschiedenen Elemente in II bestimmt wird,

ist und wobei das Reaktionsgasausgangsgemisch aus ≥50 Vol.-% Propan, ≥15 Vol.-% $O_2$ und 0 bis 35 Vol.-% Inertgas besteht und die Katalysatorschüttungen A, B in der Abfolge erst A, dann B, durchströmt.

Bevorzugte Multimetalloxide I sind demnach solche der allgemeinen Formel I'

$$[\text{Co, Ni u./o. Mg}]_a \, \text{Mo}_{1-b} \, [\text{Sn, W, P, Sb u./o. Cr}]_b \, O_x \tag{I'},$$

mit

a = 0,5 bis 1,5, vorzugsweise 0,7 bis 1,2, besonders bevorzugt 0,9 bis 1,0,

b = 0 bis 0,5, vorzugsweise >0 bis 0,5 und besonders bevorzugt 0,01 bis 0,3 sowie

x eine Zahl, die durch die Wertigkeit und Häufigkeit der von Sauerstoff verschiedenen Elemente I' bestimmt wird.

Besonders bevorzugte Multimetalloxide I sind solche der allgemeinen Formel I''

$$[\text{Co u./o. Ni}]_a \, \text{Mo}_{1-b} \, [\text{W, Sn u./o. Sb}]_b \, O_x \tag{I''},$$

mit den vorstehenden Bedeutungen für a, b und x.

Bevorzugte Multimetalloxide II sind solche der allgemeinen Formel II'

$$\text{Bi}_a \, \text{Mo}_b \, \text{W}_c \, [\text{Co,Niu./o.Zn}]_d \, \text{Fe}_e \, [\text{Pu./o.Sb}]_{f'} \, [\text{Ku./o.Na}]_g \, X^6{}_h \, O_x, \tag{II'},$$

wobei $X^6$ und die stöchiometrischen Koeffizienten die Bedeutung gemäß der allgemeinen Formel II besitzen.

Besonders bevorzugte Multimetalloxide II' sind jene mit $X^6$ = Si, Zr, Al, Nb, Ag u./o. Ti, unter denen wiederum jene mit $X^6$ = Si bevorzugt sind.

Ferner ist es günstig, wenn e' 0,5 bis 10 ist, was insbesondere dann gilt, wenn $X^6$ = Si.

Vorgenanntes gilt vor allem dann, wenn die Multimetalloxidmassen II' gemäß der EP-B 575897 hergestellt werden.

Besonders vorteilhafte Katalysatorschüttungspaare A, B sind die Kombinationen I', II' sowie I'', II'. Dies gilt vor allem dann, wenn $X^6$ = Si und e' = 0,5 bis 10.

Mit Vorteil wird bei dem erfindungsgemäßen Verfahren das Reaktionsgasausgangsgemisch bei einer Temperatur von 325 bis 480 bzw. 450°C, vorzugsweise bei 350 bis 420°C und besonders bevorzugt bei 350 bis 400°C über den aus Katalysatorschüttungen A, B bestehenden Festbettkatalysator geführt. Im Normalfall weisen die Katalysatorschüttungen A, B identische Temperaturen auf.

Handelt es sich bei dem verwendeten Katalysatorschüttungspaar A,B um eine Kombination I', II' oder I'', II', beträgt die Reaktionstemperatur in beiden Schüttungen mit Vorteil 350 bis 420, häufig 350 bis 400°C.

Ferner umfaßt das Reaktionsgasausgangsgemisch mit Vorteil ≤30 Vol.-%, vorzugsweise ≤20 Vol.-% und besonders bevorzugt ≤10 Vol.-% bzw. ≤5 Vol.-% Inertgas. Selbstverständlich kann das Reaktionsgasausgangsgemisch auch kein Inertgas umfassen. Unter Inertgas werden hier solche Gase verstanden, deren Umsatz beim Durchgang durch den erfindungsgemäß zu verwendenden Festbettkatalysator ≤5 mol-% beträgt. Als Inertgas kommen z.B. $H_2O$, $CO_2$, CO, $N_2$ und/oder Edelgase in Betracht.

Weiterhin enthält das Reaktionsgasausgangsgemisch zweckmäßig ≥60 Vol.-%, oder ≥70 Vol.-%, oder ≥80 Vol.-% Propan. Generell liegt der Propangehalt des erfindungsgemäß einzusetzenden Reaktionsgasausgangsgemisches bei ≤85 Vol.-%, häufig bei <83 oder ≤82 oder ≤81 oder ≤80 Vol.-%. Der Gehalt des Reaktionsgasausgangsgemisches an molekularem Sauerstoff kann beim erfindungsgemäßen Verfahren bis zu 35 Vol.-% betragen. Mit Vorteil liegt er bei wenigstens 20 Vol.-% oder bei wenigstens 25 Vol.-%. Erfindungsgemäß günstige Reaktionsgasausgangsgemische enthalten ≥65 Vol.-% und ≤85 Vol.-% Propan sowie ≥15 Vol.-% und ≤35 Vol.-% molekularen Sauerstoff. Erfindungsgemäß von Vorteil (im Hinblick auf Selektivität und Umsatz) ist, wenn das Molverhältnis von Propan zu molekularem Sauerstoff im Reaktionsausgangsgemisch <5:1, bevorzugt ≤4,75:1, besser ≤4,5:1 und besonders bevorzugt ≤4:1 beträgt. In der Regel wird vorgenanntes Verhältnis ≥1:1 bzw. ≥2:1 betragen.

Prinzipiell können erfindungsgemäß geeignete Aktivmassen I in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und diese bei Temperaturen von 450 bis 1000°C calciniert. Die Calcination kann sowohl unter Inertgas als auch unter einer oxidativen Atmosphäre wie z. B. Luft (Gemisch aus Inertgas und Sauerstoff) sowie auch unter reduzierender Atmosphäre (z.B. Gemisch aus Inertgas, Sauerstoff und $NH_3$, CO und/oder $H_2$) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexsalze, Ammonium-Salze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oder Ammoniumoxalat, die spätestens beim späteren Calcinieren zu vollständig gasförmig entweichenden Verbindungen zerfallen und/oder zersetzt werden können, können in das innige Trockengemisch zusätzlich eingearbeitet werden). Das innige Vermischen der Ausgangsverbindungen zur Herstellung von Multimetalloxidmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wäßrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt. Besonders geeignete Ausgangsverbindungen des Mo, V, W und Nb sind deren Oxoverbindungen (Molybdate, Vanadate, Wolframate und Niobate) bzw. die von diesen abgeleiteten Säuren. Dies gilt insbesondere für die entsprechenden Ammoniumverbindungen (Ammoniummolybdat, Ammoniumvanadat, Ammoniumwolframat).

Die Multimetalloxidmassen I können für das erfindungsgemäße Verfahren sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie er z.B. aus der DE-A 2909671 oder aus der EP-A 293859 bekannt ist. Zweckmäßigerweise kann zur Beschichtung der Trägerkörper die aufzubringende Pulvermasse befeuchtet und nach dem Aufbringen, z.B. mittels heißer Luft, wieder getrocknet werden. Die Schichtdicke der auf den Trägerkörper aufgebrachten Pulvermasse wird zweckmäßigerweise im Bereich 50 bis 500 µm, bevorzugt im Bereich 150 bis 250 µm liegend, gewählt.

Als Trägermaterialien können dabei übliche poröse oder unporöse Aluminiumoxide, Siliciumdioxid, Thoriumdioxid, Zirkondioxid, Siliciumcarbid oder Silicate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 8 mm, bevorzugt 4 bis 5 mm beträgt.

Bezüglich der Herstellung der Multimetalloxidmassen II gilt das für die Multimetalloxidmassen I Gesagte. Allerdings beträgt die angewandte Calcinationstemperatur in der Regel 350 bis 650°C. Besonders bevorzugte Multimetalloxidmassen II sind die in der EP-B 575897 offenbarten Multimetalloxidmassen I, insbesondere deren bevorzugte Ausführungsvarianten. Sie sind dadurch charakterisiert, daß zunächst aus Teilmengen der elementaren Konstituenten Multimetalloxide vorgebildet und im weiteren Herstellungsverlauf als Elementquelle eingesetzt werden.

In anwendungstechnisch zweckmäßiger Weise erfolgt die Durchführung des erfindungsgemäßen Verfahrens in Rohrbündelreaktoren wie sie z.B. in der EP-A 700893 und in der EP-A 700714 beschrieben sind. In den Metallrohren (in der Regel aus Edelstahl) befindet sich der erfindungsgemäß zu verwendende Festbettkatalysator und um die Metallrohre wird ein Temperiermedium, in der Regel eine Salzschmelze, geführt. D.h., in einfachster Weise enthält jedes Reaktionsrohr die beiden erfindungsgemäß zu verwendenden Katalysatorschüttungen A, B unmittelbar aufeinanderfolgend angeordnet. Das Verhältnis der Schüttvolumina der beiden Katalysatorschüttungen A, B beträgt erfindungsgemäß vorteilhaft 1:10 bis 10:1, vorzugsweise 1:5 bis 5:1 und besonders bevorzugt 1:2 bis 2:1, häufig 1:1. Der Reaktionsdruck beträgt im allgemeinen ≥0,5 bar. Im Regelfall wird der Reaktionsdruck 100 bar nicht überschreiten, d.h. ≥0,5 bis 100 bar betragen. Zweckmäßig beträgt Reaktionssdruck häufig >1 bis 50 bzw. >1 bis 20 bar. Bevorzugt liegt der Reaktionsdruck bei ≥1,25 bzw. ≥1,5 oder ≥1,75 bzw. ≥2 bar. Häufig wird dabei die Obergrenze von 10 bzw. 20 bar nicht überschritten. Selbstverständlich kann der Reaktionsdruck auch 1 bar betragen (vorstehende Aussagen bezüglich des Reaktionsdruckes gelten für das erfindungsgemäße Verfahren ganz generell). Ferner wird die Belastung mit Vorteil so gewählt, daß die Verweilzeit des Reaktionsgasgemisches über die beiden Katalysatorschüttungen A, B 0,5 bis 20 sec, bevorzugt 1 bis 10 sec, besonders bevorzugt 1 bis 4 sec und häufig 3 sec beträgt. Im Produktgemisch des erfindungsgemäßen Verfahrens enthaltenes Propan und/oder Propen kann abgetrennt und in die erfindungsgemäße Gasphasenoxidation rückgeführt werden. Ferner kann sich an das erfindungsgemäße Verfahren eine weitere heterogen katalysierte Oxidationsstufe anschließen, wie sie für die heterogen katalysierte Gasphasenoxidation von Acrolein zu Acrylsäure bekannt sind, in die das Produktgemisch des erfindungsgemäßen Verfahrens, gegebenenfalls unter Zusatz von weiterem molekularem Sauerstoff, überführt wird. Im Anschluß daran kann wiederum noch nicht umgesetztes Propan, Propen und/oder Acrolein abgetrennt und in die Gasphasenoxidationen rückgeführt werden.

Die Abtrennung des gebildeten Acrolein und/oder Acrylsäure aus den Produktgasgemischen kann in an sich bekannter Weise erfolgen. In der Regel beträgt der mit dem erfindungsgemäßen Verfahren erzielte Propanumsatz ≥5 mol%, bzw. ≥7,5 mol.-%. Normalerweise werden jedoch keine Propanumsätze ≥20 mol-% erzielt. Das erfindungsgemäße Verfahren eignet sich insbesondere für eine kontinuierliche Durchführung. Bei Bedarf kann auf der Höhe der Katalysatorschüttung B noch zusätzlicher molekularer Sauerstoff zugedüst werden. Im übrigen sind in dieser Schrift Umsatz, Selektivität und Verweilzeit, falls nichts anderes erwähnt wird, wie folgt definiert:

$$\text{Umsatz an Propan (mol-\%)} = \frac{\text{Molzahl umgesetztes Propan}}{\text{Molzahl eingesetztes Propan}} \times 100$$

$$\text{Selektivität S der Acrolein und/oder Acrylsäurebildung (mol-\%)} = \frac{\text{Molzahl Propan umgesetzt zu Acrolein und/oder Acrylsäure}}{\text{Molzahl umgesetztes Propan}} \times 100$$

$$\text{Verweilzeit (sec)} = \frac{\text{mit Katalysator gefülltes Leervolumen des Reaktors (l)}}{\text{durchgesetzte Menge Reaktionsgasausgangsgemisch}} \times 3600$$

EP 1 025 073 B1

Beispiele

Beispiel 1

a) Herstellung einer Multimetalloxidmasse I

In 1,2 kg Wasser wurden bei 80°C 292,4 g Ammoniumheptamolybdat (81,5 Gew.-% $MoO_3$) gelöst und zu der resultierenden Lösung 742,4 g wäßrige Cobaltnitratlösung (auf die Lösung bezogen 12,5 Gew.-% Co) zugegeben. Die entstandene Lösung wurde unter Rühren auf dem Wasserbad bei 100°C eingedampft, bis eine pastöse Masse entstanden war. Diese wurde in einem Trockenschrank 16 h bei 110°C getrocknet und anschließend in einem luftdurchströmten Muffelofen (60 l Innenvolumen, Luftdurchsatz 500 l/h) wie folgt calciniert: Zunächst wurde mit einer Aufheizrate von 120°C/h von Raumtemperatur (25°C) auf 300°C aufgeheizt. Anschließend wurde die Temperatur von 300°C während 3 h aufrechterhalten und danach wurde mit einer Aufheizrate von 125°C/h die Calcinationstemperatur von 300 auf 550°C erhöht. Diese Temperatur wurde anschließend während 6 h aufrechterhalten. Das so erhaltene Multimetalloxid wurde zerkleinert und als katalytisch aktive Multimetalloxidmasse I der Stöchiometrie $Mo_1Co_{0,95}O_x$ die Kornfraktion mit einem Korngrößtdurchmesser von 0,6 bis 1,2 mm durch Sieben abgetrennt.

b) Herstellung einer Multimetalloxidmasse II

1. Herstellung einer Ausgangsmasse

50 kg einer Lösung von $Bi(NO_3)_3$ in wäßriger Salpetersäure (11 Gew.-% Bi, 6,4 Gew.-% $HNO_3$, jeweils auf die Lösung bezogen) wurde mit 13,7 kg Ammoniumparawolframat (89 Gew.-% $WO_3$) versetzt und 1 h bei 50°C gerührt. Die erhaltene Suspension wurde sprühgetrocknet und 2 h bei 750°C calciniert. Das so erhaltene vorgebildete calcinierte Mischoxid wurde gemahlen und die Kornfraktion 1 µm $\leq$ d $\leq$ 5 µm (d= Korngrößtdurchmesser) ausklassiert. Anschließend wurde die Kornfraktion mit 1 % ihres Gewichtes an feinteiligem (zahlenmittlerer Größtdurchmesser 28 nm) $SiO_2$ vermengt.

2. Herstellung einer Ausgangsmasse 2

In 104,6 kg Co-Nitratlösung (12,5 Gew.-% Co auf die Lösung bezogen) wurden 48,9 kg $Fe(NO_3)_3$ gelöst. Die resultierende Lösung wurde zu einer Lösung von 85,5 kg Ammoniumheptamolybdat (81,5 Gew.-% $MoO_3$) in 240 l Wasser gegeben. Das resultierende Gemisch wurde mit 7,8 kg einer 20 % ihres Gewichtes kolloidales $SiO_2$ enthaltenden wäßrigen Mischung und 377 g einer 48 Gew.-% KOH enthaltenden wäßrigen Lösung versetzt. Anschließend wurde 3 h gerührt und die dabei resultierende wäßrige Suspension zur Ausgangsmasse 2 sprühgetrocknet.

3. Herstellung des Multimetalloxids II

Die Ausgangsmasse 1 wurde mit der Ausgangsmasse 2 in der für ein Multimetalloxid II der Stöchiometrie

$$Mo_{12}W_2Bi_1Co_{5,5}Fe_3Si_{1,6}K_{0,08}O_x,$$

erforderlichen Menge vermischt und zu Hohlzylindern mit 3 mm Länge, 5 mm Außendurchmesser und 1,5 mm Wandstärke verpreßt und anschließend wie folgt calciniert. Die Calcination erfolgte im Luft durchströmten Muffelofen (60 l Innenvolumen, 1 l/h Luft pro Gramm Katalysatorvorläufermasse). Mit einer Aufheizrate von 180°C/h wurde zunächst von Raumtemperatur (25°C) auf 190°C aufgeheizt. Diese Temperatur wurde für 1 h aufrechterhalten und dann mit einer Aufheizrate von 60°C/h auf 220°C erhöht. Die 220°C wurden wiederum während 2 h aufrechterhalten, bevor sie mit einer Aufheizrate von 60°C/h auf 260°C erhöht wurde. Die 260°C wurden anschließend während 1 h aufrechterhalten. Danach wurde zunächst auf Raumtemperatur abgekühlt und damit die Zersetzungsphase im wesentlichen abgeschlossen. Dann wurde mit einer Aufheizrate von 180°C/h auf 465°C erhitzt und diese Calcinationstemperatur während 4 h aufrechterhalten.
200 g der resultierenden Aktivmasse wurden zerkleinert und die Kornfraktion 0,6 bis 1,2 mm als Multimetalloxidaktivmasse II ausgesiebt.

c) Gasphasenkatalytische Oxidation von Propan

Ein Reaktionsrohr (V2A Stahl; 2,5 cm Wandstärke; 8,5 mm Innendurchmesser; elektrisch beheizt) der Länge 1,4 m wurde von unten nach oben auf einem Kontaktstuhl (7 cm Länge) zunächst auf einer Länge von 13 cm mit Quarzsplitt (zahlenmittlerer Größtdurchmesser 1 bis 2 mm) und anschließend auf einer Länge von 42,5 cm mit der Multimetalloxidaktivmasse II sowie daran anschließend auf einer Länge von 42,5 cm mit der Multimetalloxidaktivmasse I beschickt, bevor die Beschickung auf einer Länge von 13 cm mit Quarzsplitt (zahlenmittlerer Größtdurchmesser 1 bis 2 mm) abgeschlossen wurde.

Das wie vorstehend beschickte Reaktionsrohr wurde auf seiner gesamten Länge auf 430°C aufgeheizt und dann mit 56 Nl/h einer Reaktionsgasausgangsmischung aus 80 Vol-% Propan und 20 Vol-% Sauerstoff von oben nach unten beschickt.

Bei einfachem Durchgang wurde ein Produktgasgemisch erhalten, das nachfolgende Charakteristik aufwies:

Propanumsatz: 10 mol-%
Selektivität der Acroleinbildung: 59 mol-%
Selektivität der Acrylsäurebildung: 14 mol-%
Selektivität der Propenbildung: 3 mol-%

Beispiel 2

a) Herstellung einer Multimetalloxidmasse I

Die Herstellung der Multimetalloxidmasse I erfolgte wie in Beispiel 1a), die abschließende Calcinationstemperatur betrug jedoch 560°C anstelle von 550°C.

b) Herstellung einer Multimetalloxidmasse II

Die Herstellung der Multimetalloxidmasse II erfolgte wie in Beispiel 1b).

c) Gasphasenkatalytische Oxidation von Propan

Ein Reaktionsrohr (V2A Stahl; 2,5 cm Wandstärke; 8,5 mm Innendurchmesser; elektrisch beheizt) der Länge 1,4 m wurde von unten nach oben auf einem Kontaktstuhl (7 cm Länge) zunächst auf einer Länge von 18 cm mit Quarzsplitt (zahlenmittlerer Größtdurchmesser 1 bis 2 mm) und anschließend auf einer Länge von 42,5 cm mit der Multimetalloxidaktivmasse II sowie daran anschließend auf einer Länge von 42,5 cm mit der Multimetalloxidaktivmasse I beschickt, bevor die Beschickung auf einer Länge von 30 cm mit Quarzsplitt (zahlenmittlerer Größtdurchmesser 1 bis 2 mm) abgeschlossen wurde.

Das wie vorstehend beschickte Reaktionsrohr wurde auf seiner gesamten Länge auf 415°C aufgeheizt und dann mit 56 Nl/h einer Reaktionsgasausgangsmischung aus 80 Vol-% Propan und 20 Vol-% Sauerstoff von oben nach unten beschickt. Der Druck am Reaktionsrohreingang betrug 1,68 bar (absolut). Der Druckabfall längs des Reaktionsrohres betrug 0,27 bar.

Bei einfachem Durchgang wurde ein Produktgasgemisch erhalten, das nachfolgende Charakteristik aufwies:

Propanumsatz: 11,5 mol-%
Selektivität der Acroleinbildung: 66 mol-%
Selektivität der Acrylsäurebildung: 10 mol-%
Selektivität der Propenbildung: 2 mol-%

Beispiel 3

a) Herstellung einer Multimetalloxidmasse I

In 3,6 kg Wasser wurden bei 45°C 877,2 g Ammoniumheptamolybdat (81,5 Gew.-% $MoO_3$) gelöst und zu der resultierenden Lösung 2227,2 g wäßrige Cobaltnitratlösung (auf die Lösung bezogen 12,5 Gew.-% Co) zugegeben.

Die entstandene klare, rotgefärbte Lösung wurde in einem Sprühtrockner der Fa. Niro bei einer Eingangstemperatur von 330 bis 340°C und einer Ausgangstemperatur von 110°C sprühgetrocknet (A/S Niro Atomizer transportable Minor-Anlage). 450 g des erhaltenen Sprühpulvers wurden während 40 min mit 75 ml $H_2O$ verknetet (1-1-Kneter vom Typ Sigmaschaufel-Kneter der Fa. Werner & Pfleiderer) und in einem Umlufttrockenschrank 16 h bei 110°C getrocknet. Anschließend wurde das getrocknete Pulver in einem von Luft durchströmten, rotierenden (15 Umdrehungen/min) Quarzrundkolben (Innenvolumen: 2 l, Luftdurchsatz: konstant 250 l/h) wie folgt calciniert (Klappofenbeheizung):

Zunächst wurde mit einer Aufheizrate von 180°C/h von Raumtemperatur (25°C) auf 225°C aufgeheizt. Anschließend wurde die Temperatur von 225°C während 0,5 h aufrechterhalten und danach wurde mit einer Aufheizrate von 60°C/h die Calcinationstemperatur von 225°C auf 300°C erhöht. Diese Temperatur wurde anschließend während 3 h aufrechterhalten. Danach wurde mit einer Aufheizrate von 125°C/h die Calcinationstemperatur von 300 auf 550°C erhöht. Diese Temperatur wurde anschließend während 6 h aufrechterhalten.

Das so erhaltene Multimetalloxid wurde zerkleinert und als katalytisch aktive Multimetalloxidmasse I der Stöchiometrie $Mo_1CO_{0,95}O_x$ die Kornfraktion mit einem Korngrößtdurchmesser von 0,6 bis 1,2 mm durch Sieben abgetrennt.

b) Herstellung einer Multimetalloxidmasse II

Die Herstellung der Multimetalloxidmasse II erfolgte wie in Beispiel 1b).

c) Gasphasenkatalytische Oxidation von Propan

Ein Reaktionsrohr (V2A Stahl; 2,5 cm Wandstärke; 8,5 mm Innendurchmesser; elektrisch beheizt) der Länge 1,4 m wurde von unten nach oben auf einem Kontaktstuhl (7 cm Länge) zunächst auf einer Länge von 18 cm mit Quarzsplitt (zahlenmittlerer Größtdurchmesser 1 bis 2 mm) und anschließend auf einer Länge von 42,5 cm mit der Multimetalloxidaktivmasse II sowie daran anschließend auf einer Länge von 42,5 cm mit der Multimetalloxidaktivmasse I beschickt, bevor die Beschickung auf einer Länge von 30 cm mit Quarzsplitt (zahlenmittlerer Größtdurchmesser 1 bis 2 mm) abgeschlossen wurde.

Das wie vorstehend beschickte Reaktionsrohr wurde auf seiner gesamten Länge auf 390°C aufgeheizt und dann mit 84 Nl/h einer Reaktionsgasausgangsmischung aus 80 Vol-% Propan und 20 Vol-% Sauerstoff von oben nach unten beschickt. Der Druck am Reaktionsrohreingang betrug 2,7 bar (absolut). Der Druckabfall längs des Reaktionsrohres betrug 0,35 bar.

Bei einfachem Durchgang wurde ein Produktgasgemisch erhalten, das nachfolgende Charakteristik aufwies:

Propanumsatz: 9,0 mol-%
Selektivität der Acroleinbildung: 69 mol-%
Selektivität der Acrylsäurebildung: 10 mol-%
Selektivität der Propenbildung: 1 mol-%

Beispiel 4

a) Herstellung einer Multimetalloxidmasse I

In 1,5 kg Wasser wurden bei 45°C 929,3 g Ammoniumheptamolybdat (81,5 Gew.-% $MoO_3$) gelöst. Ferner wurden in 1,5 kg Wasser bei 95°C bis 98°C 80,8 g Ammoniumparawolframat (89,04 Gew.-% $Wo_3$) gelöst und anschließend auf 45°C abgekühlt. Beide Lösungen wurden bei 45°C vereinigt und mit einer ebenfalls 45°C aufweisenden wäßrigen Cobaltnitratlösung (auf die Lösung bezogen 12,5 Gew.-% Co) versetzt.

Die dabei entstandene klare, rotgefärbte Lösung wurde in einem Sprühtrockner der Fa. Niro bei einer Eingangstemperatur von 320°C und einer Ausgangstemperatur von 110°C sprühgetrocknet (A/S Niro Atomizer transportable Minor-Anlage). 450 g des erhaltenen Sprühpulvers wurden während 40 min mit 85 ml $H_2O$ verknetet (1-l-Kneter vom Typ Sigmaschaufel-Kneter der Fa. Werner & Pfleiderer) und in einem Umlufttrockenschrank 16 h bei 110°C getrocknet. Anschließend wurde die getrocknete Masse in einem luftdurchströmten Muffelofen (Innenvolumen: 60 l, Luftdurchsatz: konstant 500 l/h) wie folgt calciniert.

Zunächst wurde mit einer Aufheizrate von 120°C/h von Raumtemperatur (25°C) auf 300°C aufgeheizt. Anschließend wurde die Temperatur von 300°C während 3 h aufrecht erhalten und danach wurde mit einer Aufheizrate von 125°C/h die Calcinationstemperatur von 300°C auf 567°C erhöht. Diese Temperatur wurde anschließend während 6 h aufrecht erhalten.

Das so erhaltene Multimetalloxid wurde zerkleinert und als katalytisch aktive Multimetalloxidmasse I der Stöchiometrie $Co_{0,95}Mo_{0,95}W_{0,05}O_x$ die Kornfraktion mit einem Korngrößtdurchmesser von 0,6 bis 1,2 mm durch Sieben abgetrennt.

b) Herstellung einer Multimetalloxidmasse II

Die Herstellung der Multimetalloxidmasse II erfolgte wie in Beispiel 1b).

c) Gasphasenkatalytische Oxidation von Propan

Ein Reaktionsrohr (V2A Stahl; 2,5 cm Wandstärke; 8,5 mm Innendurchmesser; elektrisch beheizt) der Länge 1,4 m wurde von unten nach oben auf einem Kontaktstuhl (7 cm Länge) zunächst auf einer Länge von 18 cm mit Quarzsplitt (zahlenmittlerer Größtdurchmesser 1 bis 2 mm) und anschließend auf einer Länge von 42,5 cm mit der Multimetalloxidaktivmasse II sowie daran anschließend auf einer Länge von 42,5 cm mit der Multimetalloxidaktivmasse I beschickt, bevor die Beschickung auf einer Länge von 30 cm mit Quarzsplitt (zahlenmittlerer Größtdurchmesser 1 bis 2 mm) abgeschlossen wurde.

Das wie vorstehend beschickte Reaktionsrohr wurde auf seiner gesamten Länge auf 395°C aufgeheizt und dann mit 98 Nl/h einer Reaktionsgasausgangsmischung aus 80 Vol-% Propan und 20 Vol-% Sauerstoff von oben nach unten beschickt. Der Druck am Reaktionsrohreingang betrug 2,69 bar (absolut). Der Druckabfall längs des Reaktionsrohres betrug 0,36 bar.

Bei einfachem Durchgang wurde ein Produktgasgemisch erhalten, das nachfolgende Charakteristik aufwies:

Propanumsatz: 8,2 mol-%
Selektivität der Acroleinbildung: 67 mol-%
Selektivität der Acrylsäurebildung: 11 mol-%
Selektivität der Propenbildung: 1 mol-%

## Patentansprüche

**1.** Verfahren der heterogen katalysierten Gasphasenoxidation von Propan zu Acrolein und/oder Acrylsäure, bei dem man ein aus Propan, molekularem Sauerstoff und gegebenenfalls Inertgas bestehendes Reaktionsgasausgangsgemisch bei einer Temperatur von 300 bis 500°C über einen Festbettkatalysator führt, **dadurch gekennzeichnet, daß** der Festbettkatalysator aus zwei räumlich aufeinanderfolgend angeordneten Katalysatorschüttungen A, B besteht, mit der Maßgabe, daß die Aktivmasse der Schüttung A wenigstens ein Multimetalloxid der allgemeinen Formel I

$$M^1_a Mo_{1-b} M^2_b O_x \qquad (I),$$

mit

$M^1 =$ Co, Ni, Mg, Zn, Mn und/oder Cu,
$M^2 =$ W, V, Te, Nb, P, Cr, Fe, Sb, Ce, Sn und/oder La,
$a =$ 0,5 bis 1,5,
$b =$ 0 bis 0,5
$x =$ eine Zahl, die durch die Wertigkeit und Häufigkeit der von Sauerstoff verschiedenen Elemente in I bestimmt wird,

und die Aktivmasse der Schüttung B wenigstens ein Multimetalloxid der allgemeinen Formel II

$$Bi_a, Mo_b, X^1_c, X^2_d, X^3_e, X^4_f, X^5_g, X^6_h, O_x, \qquad (II),$$

mit

$X^1 =$ W, V und/oder Te,
$X^2 =$ Erdalkalimetall, Co, Ni, Zn, Mn, Cu, Cd, Sn und/oder Hg,
$X^3 =$ Fe, Cr und/oder Ce,
$X^4 =$ P, As, Sb und/oder B,
$X^5 =$ Alkalimetall, Tl und/oder Sn,
$X^6 =$ seltenes Erdmetall, Ti, Zr, Nb, Ta, Re, Ru, Rh, Ag, Au, Al, Ga, In, Si, Ge, Th und/oder U,
$a' =$ 0,01 bis 8,
$b' =$ 0,1 bis 30,
$c' =$ 0 bis 20,

d'= 0 bis 20,

e'= 0 bis 20,

f'= 0 bis 6,

g'= 0 bis 4,

h'= 0 bis 15,

x'= eine Zahl, die durch die Wertigkeit und Häufigkeit der von Sauerstoff verschiedenen Elemente in II bestimmt wird,

ist, und wobei das Reaktionsgasausgangsgemisch aus ≥50 Vol.-% Propan, ≥15 Vol.-% $O_2$ und 0 bis 35 Vol.-% Inertgas besteht und die Katalysatorschüttungen A, B in der Abfolge erst A, dann B, durchströmt.

2. Verfahren nach Anspruch 1, **dadurch gekennzeichnet, daß** die Temperatur 325 bis 450°C beträgt.

3. Verfahren nach Anspruch 1, **dadurch gekennzeichnet, daß** die Temperatur 350 bis 420°C beträgt.

4. Verfahren nach einem der Ansprüche 1 bis 3, **dadurch gekennzeichnet, daß** das Reaktionsgasausgangsgemisch ≥60 Vol.-% Propan enthält.

5. Verfahren nach einem der Ansprüche 1 bis 3, **dadurch gekennzeichnet, daß** das Reaktionsgasausgangsgemisch ≥70 Vol. -% Propan enthält.

6. Verfahren nach einem der Ansprüche 1 bis 5, **dadurch gekennzeichnet, daß** das Reaktionsgasausgangsgemisch ≥20 Vol.-% $O_2$ enthält.

7. Verfahren nach einem der Ansprüche 1 bis 6, **dadurch gekennzeichnet, daß** man es kontinuierlich durchführt.

8. Verfahren nach einem der Ansprüche 1 bis 7, **dadurch gekennzeichnet, daß** das Molverhältnis von Propan zu molekularem Sauerstoff im Reaktionsgasausgangsgemisch <5 beträgt.

9. Verfahren nach einem der Ansprüche 1 bis 8, **dadurch gekennzeichnet, daß** das Verhältnis der Schüttvolumina der beiden Katalysatorschüttungen A, B 1:5 bis 5:1 beträgt.

10. Verfahren nach einem der Ansprüche 1 bis 9, **dadurch gekennzeichnet, daß** der Reaktionsdruck >1 bar beträgt.

## Claims

1. A process for the heterogeneously catalyzed gas-phase oxidation of propane to acrolein and/or acrylic acid, in which a reaction gas starting mixture comprising propane, molecular oxygen and, if desired, inert gas is passed at from 300 to 500°C over a fixed-bed catalyst which comprises two catalyst beds A and B arranged spatially in succession, with the proviso that the active composition of bed A is at least one multimetal oxide of the formula I

$$M^1_a Mo_{1-b} M^2_b O_x \qquad (I),$$

where

$M^1$ = Co, Ni, Mg, Zn, Mn and/or Cu,

$M^2$ = W, V, Te, Nb, P, Cr, Fe, Sb, Ce, Sn and/or La,

a = 0.5 - 1.5,

b = 0 - 0.5

x = a number which is determined by the valence and amount of the elements different from oxygen in I,

and the active composition of bed B is at least one multimetal oxide of the formula II

$$Bi_a, Mo_b, X^1_c, X^2_d, X^3_e, X^4_f, X^5_g, X^6_h, O_x, \qquad (II),$$

where

$X^1$ = W, V and/or Te,
$X^2$ = alkaline earth metal, Co, Ni, Zn, Mn, Cu, Cd, Sn and/or Hg,
$X^3$ = Fe, Cr and/or Ce,
$X^4$ = P, As, Sb and/or B,
$X^5$ = alkali metal, Tl and/or Sn,
$X^6$ = rare earth metal, Ti, Zr, Nb, Ta, Re, Ru, Rh, Ag, Au, Al, Ga, In, Si, Ge, Th and/or U,
a' = 0.01 - 8,
b' = 0.1 - 30,
c' = 0 - 20,
d' = 0 - 20,
e' = 0 - 20,
f' = 0 - 6,
g' = 0 - 4,
h' = 0 - 15,
x' = a number which is determined by the valence and amount of the elements different from oxygen in II,

wherein the reaction gas starting mixture comprises $\geq$50% by volume of propane, $\geq$15% by volume of $O_2$ and from 0 to 35% by volume of inert gas and flows through the catalyst beds A and B in the order first A, then B.

2. A process as claimed in claim 1, wherein the temperature is from 325 to 450°C.

3. A process as claimed in claim 1, wherein the temperature is from 350 to 420°C.

4. A process as claimed in any of claims 1 to 3, wherein the reaction gas starting mixture comprises $\geq$60% by volume of propane.

5. A process as claimed in any of claims 1 to 3, wherein the reaction gas starting mixture comprises $\geq$70% by volume of propane.

6. A process as claimed in any of claims 1 to 5, wherein the reaction gas starting mixture comprises $\geq$20% by volume of $O_2$.

7. A process as claimed in any of claims 1 to 6 which is carried out continuously.

8. A process as claimed in any of claims 1 to 7, wherein the molar ratio of propane to molecular oxygen in the reaction gas starting mixture is <5.

9. A process as claimed in any of claims 1 to 8, wherein the ratio of the bed volumes of the two catalyst beds A, B is from 1:5 to 5:1.

10. A process as claimed in any of claims 1 to 9, wherein the reaction pressure is >1 bar.

**Revendications**

1. Procédé d'oxydation en phase gazeuse sous catalyse hétérogène du propane en acroléine et/ou acide acrylique, où l'on fait passer sur un catalyseur à lit fixe un mélange réactionnel de départ constitué de propane, d'oxygène moléculaire et éventuellement d'un gaz inerte, à une température de 300 à 500°C, **caractérisé en ce que** le catalyseur à lit fixe est constitué de deux remplissages de catalyseurs A, B, spatialement successifs, avec la condition que la masse active du remplissage A soit au moins un oxyde métallique multiple de la formule générale I :

$$M^1{}_a Mo_{1-b} M^2{}_b O_x \qquad\qquad (I)$$

avec

$M^1 =$ Co, Ni, Mg, Zn, Mn et/ou Cu,

$M^2 =$ W, V, Te, Nb, P, Cr, Fe, Sb, Ce, Sn et/ou La,

a = 0,5 à 1,5,

b = 0 à 0,5,

x = un nombre déterminé par la valence et la fréquence des éléments différents de l'oxygène dans I,

et que la masse active du remplissage B soit au moins un oxyde métallique multiple de la formule générale II :

$$Bi_a, Mo_b, X^1_c, X^2_d, X^3_e, X^4_f, X^5_g, X^6_h, O_x \qquad (II)$$

avec

$X^1 =$ W, V et/ou Te,

$X^2 =$ métal alcalino-terreux, Co, Ni, Zn, Mn, Cu, Cd, Sn et/ou Hg,

$X^3 =$ Fe, Cr et/ou Ce,

$X^4 =$ P, As, Sb et/ou B,

$X^5 =$ métal alcalin, Tl et/ou Sn,

$X^6 =$ terre rare, Ti, Zr, Nb, Ta, Re, Ru, Rh, Ag, Au, Al, Ga, In, Si, Ge, Th et/ou U,

a' = 0,01 à 8,

b' = 0,1 à 30,

c'= 0 à 20,

d'= 0 à 20,

e'= 0 à 20,

f' = 0 à 6,

g' = 0 à 4,

h'= 0 à 15,

x' = un nombre déterminé par la valence et la fréquence des éléments différents de l'oxygène dans II,

et où le mélange réactionnel de départ est constitué de $\geq 50\%$ en volume de propane, $\geq 15\%$ en volume de $O_2$ et de 0 à 35% en volume de gaz inerte, et passe sur les remplissages de catalyseur A, B en séquence sur A d'abord et sur B ensuite.

2.  Procédé selon la revendication 1, **caractérisé en ce que** la température est de 325 à 450°C.

3.  Procédé selon la revendication 1, **caractérisé en ce que** la température est de 350 à 420°C.

4.  Procédé selon l'une des revendications 1 à 3, **caractérisé en ce que** le mélange réactionnel de départ contient $\geq 60\%$ en volume de propane.

5.  Procédé selon l'une des revendications 1 à 3, **caractérisé en ce que** le mélange réactionnel de départ contient $\geq 70\%$ en volume de propane.

6.  Procédé selon l'une des revendications 1 à 5, **caractérisé en ce que** le mélange réactionnel de départ contient $\geq 20\%$ en volume de $O_2$.

7.  Procédé selon l'une des revendications 1 à 6, **caractérisé en ce qu'**il est exécuté en continu.

8.  Procédé selon l'une des revendications 1 à 7, **caractérisé en ce que** le rapport molaire du propane à l'oxygène moléculaire dans le mélange réactionnel de départ est inférieur à 5.

9.  Procédé selon l'une des revendications 1 à 8, **caractérisé en ce que** le rapport des volumes apparents des deux remplissages de catalyseur A, B est de 1:5 à 5:1.

10. Procédé selon l'une des revendications 1 à 9, **caractérisé en ce que** la pression de réaction est supérieure à 1 bar.